(19) Europäisches Patentamt
European Patent Office
Office européen des brevets

(11) Veröffentlichungsnummer: **0 458 111 A1**

(12) **EUROPÄISCHE PATENTANMELDUNG**

(21) Anmeldenummer: **91107256.9**

(51) Int. Cl.5: **C07D 253/06**, A01N 43/707

(22) Anmeldetag: **04.05.91**

(30) Priorität: **19.05.90 DE 4016158**

(43) Veröffentlichungstag der Anmeldung:
**27.11.91 Patentblatt 91/48**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Findeisen, Kurt, Dr.**
**Dünfelder Strasse 28**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **1,6-Dihydro-3-dimethylamino-4-methyl-1,2,4-triazin-Derivate.**

(57) Die Erfindung betrifft neue 1,6-Dihydro-3-dimethylamino-4-methyl-1,2,4-triazin-Derivate der Formel (I),

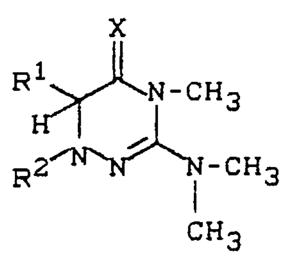

(I)

in welcher

$R^1$ für Alkyl, Cycloalkyl, für gegebenenfalls substituiertes Aralkyl oder für gegebenenfalls substituiertes Aryl steht,

$R^2$ für Wasserstoff oder für einen Rest

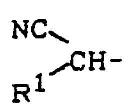

steht, wobei $R^1$ die oben angegebene Bedeutung hat, und

X für Sauerstoff oder eine NH-Gruppe steht,

verschiedene Verfahren zu ihrer Herstellung und ihre Verwendung als Herbizide.

EP 0 458 111 A1

Die Erfindung betrifft neue 1,6-Dihydro-3-dimethylamino-4-methyl-1,2,4-triazin-Derivate, verschiedene Verfahren zu ihrer Herstellung und ihre Verwendung als Herbizide.

Es ist bekannt, daß bestimmte 1,2,4-Triazin-Derivate, wie beispielsweise die Verbindung 3-Dimethylamino-4-methyl-6-cyclohexyl-1,2,4-triazin-5(4H)-on oder die Verbindung 3-Dimethylamino-4-methyl-6-isopropyl-1,2,4-triazin-5(4H)-on herbizide Eigenschaften besitzen (vergleiche z. B. EP 15 452 sowie EP 168 352).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 1,6-Dihydro-3-dimethylamino-4-methyl-1,2,4-triazin-Derivate der allgemeinen Formel (I),

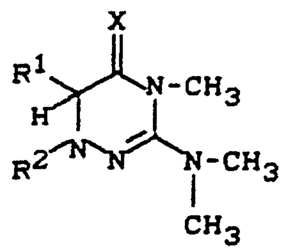

(I)

in welcher

$R^1$ für Alkyl, Cycloalkyl, für gegebenenfalls substituiertes Aralkyl oder für gegebenenfalls substituiertes Aryl steht,

$R^2$ für Wasserstoff oder für einen Rest

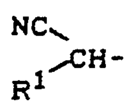

steht, wobei $R^1$ die oben angegebene Bedeutung hat, und

X für Sauerstoff oder eine NH-Gruppe steht,

gefunden.

Weiterhin wurde gefunden, daß man die neuen 1,6-Dihydro-3-dimethylamino-4-methyl-1,2,4-triazin-Derivate der allgemeinen Formel (I),

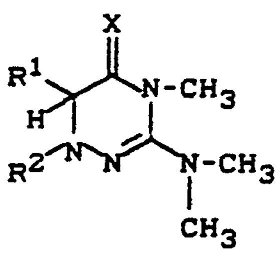

(I)

in welcher

$R^1$ für Alkyl, Cycloalkyl, für gegebenenfalls substituiertes Aralkyl oder für gegebenenfalls substituiertes Aryl steht,

$R^2$ für Wasserstoff oder für einen Rest

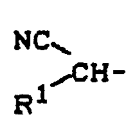

steht, wobei $R^1$ die oben angegebene Bedeutung hat, und

X für Sauerstoff oder eine NH-Gruppe steht,

nach einem der im folgenden beschriebenen Verfahren erhält.

(A) Man erhält 1,6-Dihydro-3-dimethylamino-4-methyl-5-imino-1,2,4-triazin-Derivate der Formel (Ia),

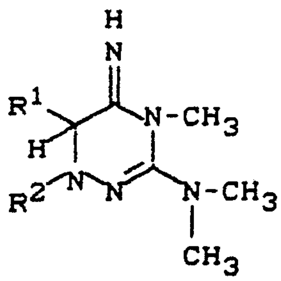

(Ia)

in welcher

$R^1$ und $R^2$ die oben angegebene Bedeutung haben,

wenn man 1-Amino-2,2,3-trimethylguanidiniumhydrochlorid der Formel (II)

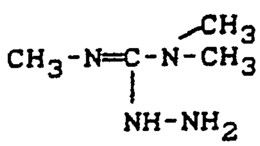

x HCl (II)

mit Cyanhydrin-Derivaten der Formel (III),

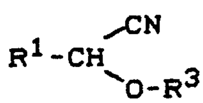

(III)

in welcher

$R^1$ die oben angegebene Bedeutung hat und

$R^3$ für Wasserstoff oder eine Acetylgruppe steht,

im Molverhältnis 1:1 oder im Molverhältnis 1:2, gegebenenfalls in Gegenwart eines Verdünnungsmittels und gegebenenfalls in Gegenwart eines Säurebindemittels umsetzt, wobei man bei einem Molverhältnis der Ausgangsstoffe (II):(III) von 1:1 Endprodukte der Formel (Ia), worin $R^2$ für H steht, und bei einem Molverhaltnis (II):(III) von 1:2 Endprodukte der Formel (Ia), worin $R^2$ für einen Rest

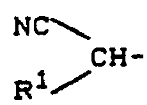

steht, erhält;

(B) man erhält 1,6-Dihydro-3-dimethylamino-4-methyl-1,2,4-triazin-5(4H)-on-Derivate der Formel (Ib),

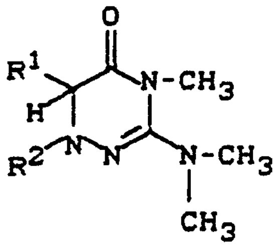

(Ib)

in welcher
$R^1$ und $R^2$ die oben angegebene Bedeutung haben,
wenn man die nach Verfahren (A) erhältlichen 1,6-Dihydro-3-dimethylamino-4-methyl-5-imino-1,2,4-triazin-Derivate der Formel (Ia),

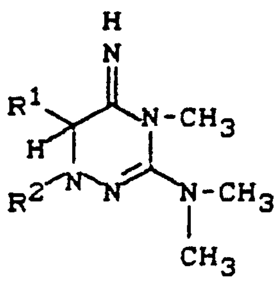

(Ia)

in welcher
$R^1$ und $R^2$ die oben angegebene Bedeutung haben,
mit wässriger Säure gegebenenfalls in Gegenwart eines Verdünnungsmittels umsetzt;
(C) man erhält 1,6-Dihydro-3-dimethylamino-4-methyl-1,2,4-triazin-5(4H)-on-Derivate der Formel (Ic),

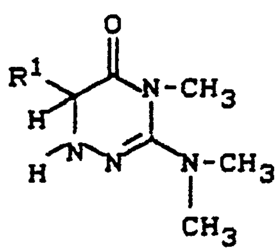

(Ic)

in welcher
$R^1$ die oben angegebene Bedeutung hat,
alternativ auch, wenn man 3-Dimethylamino-4-methyl-1,2,4-triazin-5(4H)-one der Formel (IV),

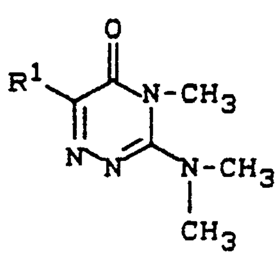

(IV)

in welcher
$R^1$ die oben angegebene Bedeutung hat,
mit Wasserstoff in Gegenwart eines Hydrierungskatalysators und gegebenenfalls in Gegenwart eines Verdünnungsmittels umsetzt.

Schließlich wurde gefunden, daß die neuen 1,6-Dihydro-3-dimethylamino-4-methyl-1,2,4-triazin-Derivate der allgemeinen Formel (I) herbizide, insbesondere auch selektivherbizide Eigenschaften besitzen.

Überraschenderweise zeigen die erfindungsgemäßen 1,6-Dihydro-3-dimethylamino-4-methyl-1,2,4-triazin-Derivate der Formel (I) neben einer deutlich verbesserten herbiziden Wirksamkeit auch eine signifikante Erhöhung der Nutzpflanzenselektivität im Vergleich zu den aus dem Stand der Technik bekannten Verbindungen 3-Dimethylamino-4-methyl-6-cyclohexyl-1,2,4-triazin-5(4H)-on oder 3-Dimethylamino-4-methyl-6-isopropyl-1,2,4-triazin-5(4H)-on, welche chemisch und wirkungsmäßig naheliegende Verbindungen sind.

Die erfindungsgemäßen 1,6-Dihydro-3-dimethylamino-4-methyl-1,2,4-triazin-Derivate sind durch die Formel (I) allgemein definiert. Bevorzugt sind Verbindungen der Formel (I), bei welchen

$R^1$ für geradkettiges oder verzweigtes Alkyl mit 1 bis 12 Kohlenstoffatomen, für Cycloalkyl mit 3 bis 8 Kohlenstoffatomen, für gegebenenfalls im Arylteil einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen steht, wobei als Arylsubstituenten jeweils infrage kommen:
Halogen, Cyano, Amino, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^2$ für Wasserstoff oder für einen Rest

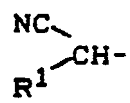

steht, wobei $R^1$ die oben angegebene vorzugsweise Bedeutung hat und

X für Sauerstoff oder eine NH-Gruppe steht.

Besonders bevorzugt sind Verbindungen der Formel (I), bei welchen

$R^1$ für geradkettiges oder verzweigtes Alkyl mit 1 bis 6 Kohlenstoffatomen, für Cycloalkyl mit 3 bis 7 Kohlenstoffatomen, für gegebenenfalls im Phenylteil ein- bis dreifach, gleich oder verschieden substituiertes Phenylalkyl mit 1 bis 4 Kohlenstoffatomen im geradkettigen oder verzweigten Alkylteil oder für gegebenenfalls ein- bis dreifach, gleich oder verschieden substituiertes Phenyl steht, wobei als Substituenten jeweils infrage kommen: Fluor, Chlor, Brom, Amino, Methyl, Ethyl, n- oder i-Propyl, Methoxy, Ethoxy, n- oder i-Propoxy, Methylthio, Trifluormethyl, Trifluormethoxy oder Trifluormethylthio,

$R^2$ für Wasserstoff oder für einen Rest

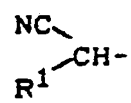

steht, wobei $R^1$ die oben angegebene besonders bevorzugte Bedeutung hat und

X für Sauerstoff oder eine NH-Gruppe steht.

Ganz besonders bevorzugt sind Verbindungen der Formel (I), bei welchen

$R^1$ für Methyl, Ethyl, n- oder i-Propyl, n-, i-, s- oder t-Butyl, für Cyclopropyl, Cyclopentyl, Cyclohexyl, für gegebenenfalls einfach oder zweifach im Phenylteil substituiertes Benzyl oder für gegebenenfalls einfach oder zweifach substituiertes Phenyl steht, wobei als Substituenten jeweils infrage kommen:
Amino, Fluor, Chlor, Brom, Methyl, Ethyl, Methoxy oder Ethoxy,

$R^2$ für Wasserstoff steht und

X für Sauerstoff oder eine NH-Gruppe steht.

Im einzelnen seien die bei den Herstellungsbeispielen aufgeführten Verbindungen genannt.

Verwendet man beispielsweise 1-Amino-2,2,3-trimethylguanidiniumhydrochlorid und Butyraldehyd-cyanhydrin in äquimolaren Mengen als Ausgangsstoffe, so läßt sich der Reaktionsablauf des erfindungsgemäßen Verfahren (A) durch das folgende Formelschema darstellen:

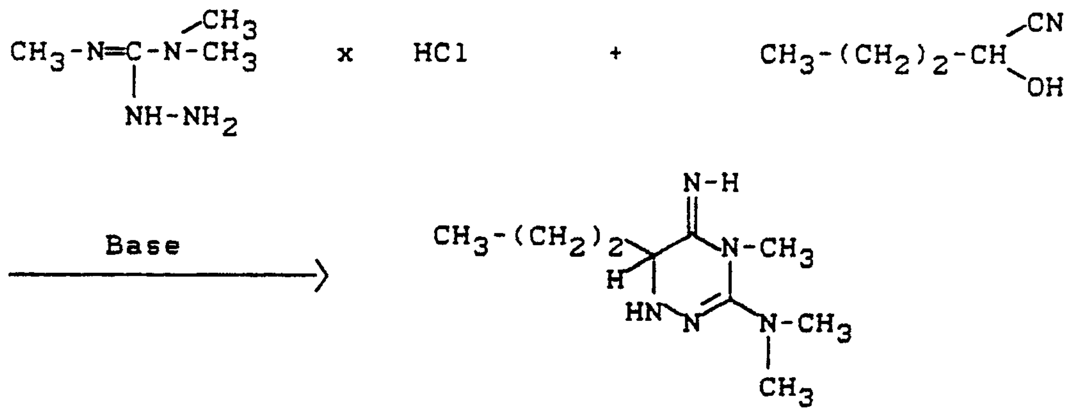


Verwendet man dagegen z.B. 1-Amino-2,2,3-trimethylguanidiniumhydrochlorid und Butyraldehyd-cyanhydrin im Molverhältnis 1:2 als Ausgangsstoffe, so kann der Reaktionsablauf nach dem erfindungsgemäßen Verfahren (A) durch das folgende Formelschema wiedergegeben werden:

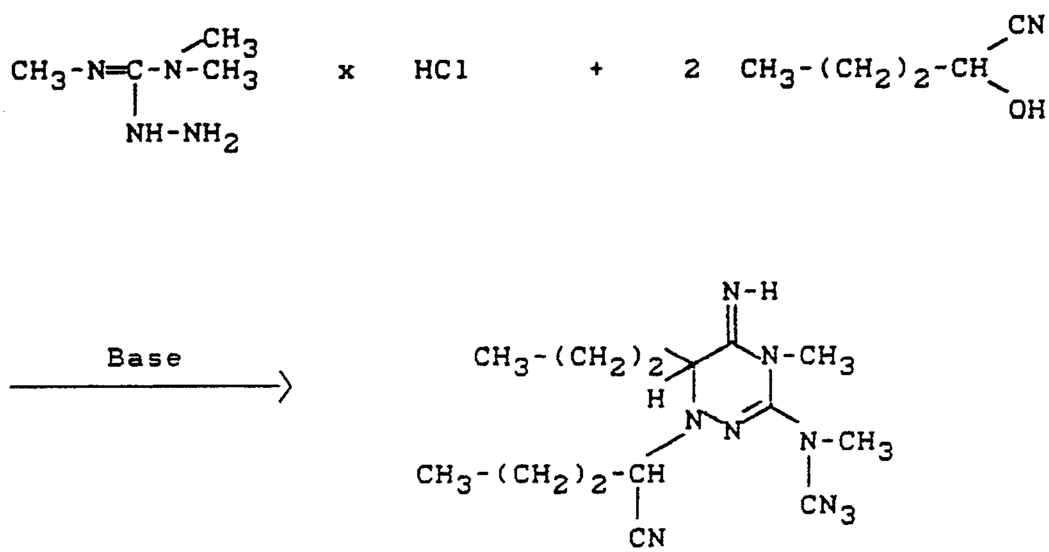


Verwendet man beispielsweise 1,6-Dihydro-3-dimethylamino-5-imino-4-methyl-6-propyl-1,2,4-triazin und verdünnte Salzsäure als Ausgangsstoffe, so läßt sich der Reaktionsablauf des erfindungsgemäßen Verfahrens (B), d.h. die saure Hydrolyse, durch das folgende Formelschema darstellen:

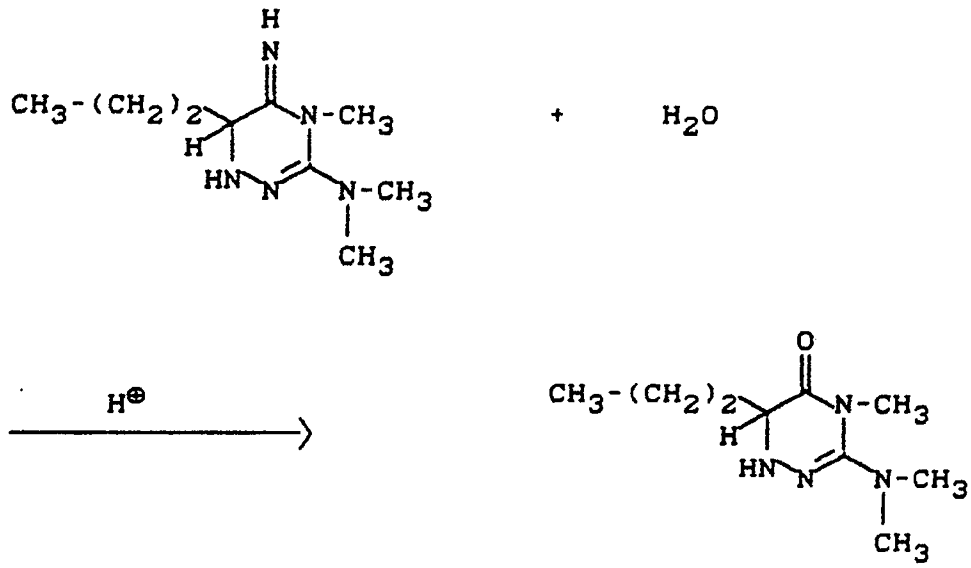


Verwendet man beispielsweise 6-t-Butyl-3-dimethylamino-4-methyl-1,2,4-triazin-5-on und molekularen Wasserstoff in Gegenwart eines Hydrierungskatalysators als Ausgangsstoffe, so läßt sich der Reaktionsablauf des erfindungsgemäßen Verfahrens (C) durch das folgende Formelschema darstellen:

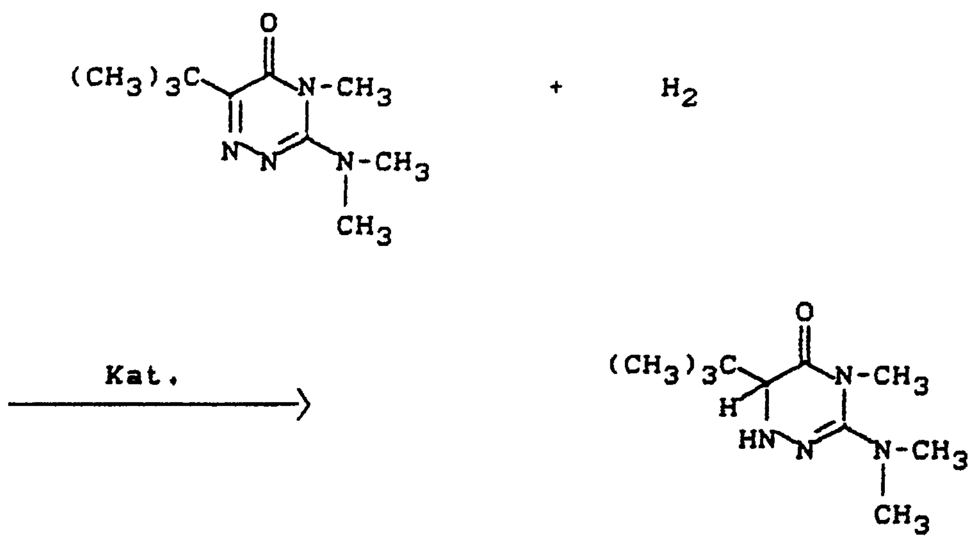


Das zur Durchführung des erfindungsgemäßen Verfahrens (A) als Ausgangsverbindung benötigte 1-Amino-2,2,3-trimethylguanidiniumhydrochlorid der Formel (II) ist bekannt (vergleiche z. B. EP 15 452 und EP 283 876 sowie Herstellungsbeispiele).

Die zur Durchführung des erfindungsgemäßen Verfahrens (A) weiterhin als Ausgangsstoffe benötigten Cyanhydrin-Derivate sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden.

$R^3$ steht vorzugsweise für Wasserstoff oder für eine Acetylgruppe.

Die Cyanhydrin-Derivate der Formel (III) sind allgemein bekannte Verbindungen der organischen Chemie (vergleiche z. B. Houben-Weyl "Methoden der organischen Chemie", Band E5, Seite 1413, 1418).

Die zur Durchführung des erfindungsgemäßen Verfahrens (B) als Ausgangsstoffe benötigten 1,6-Dihydro-3-dimethylamino-4-methyl-1,2,4-triazin-Derivate sind durch die Formel (Ia) allgemein definiert. In dieser Formel (Ia) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1,6-Dihydro-3-dimethylamino-4-methyl-1,2,4-triazin-Derivate der Formel (Ia) sind erfindungsgemäße

Verbindungen und erhältlich mit Hilfe des erfindungsgemäßen Verfahrens (A).

Die zur Durchführung des erfindungsgemäßen Verfahrens (C) als Ausgangsstoffe benötigten 3-Dimethylamino-4-methyl-1,2,4-triazin-5-one sind durch die Formel (IV) allgemein definiert. In dieser Formel (IV) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die 3-Dimethylamino-4-methyl-1,2,4-triazin-5(4H)-one der Formel (IV) sind bekannt oder erhältlich mit Hilfe bekannter Verfahren (vergleiche z. B. EP 15 452; DE 29 08 963; DE 29 08 964; DE 29 38 384; DE 33 23 953; EP 168 352).

Als Verdünnungsmittel zur Durchführung des erfindungsgemäßen Verfahrens (A) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Hexan, Cyclohexan, Dichlormethan, Chloroform, Tetrachlorkohlenstoff, Dioxan, Tetrahydrofuran oder Ethylenglykoldimethyl- oder -diethylether, Nitrile wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Methanol, Ethanol, n- oder i-Propanol sowie n- oder i-Butanol.

Das erfindungsgemäße Verfahren (A) wird vorzugsweise in Gegenwart eines geeigneten Säurebindemittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (A) in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung des erfindungsgemäßen Verfahrens (A) setzt man - wie bereits angegeben - 1-Amino-2,2,3-trimethylguanidinium-hydrochlorid der Formel (II) und Cyanhydrin-Derivate der Formel (III) in praktisch äquimolaren Mengen um, wenn Endprodukte der Formel (I) mit $R^2$ = H hergestellt werden sollen.

Wünscht man dagegen Endprodukte der Formel (I), worin $R^2$ für einen Rest

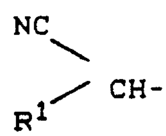

steht, herzustellen, so setzt man pro Mol 1-Amino-2,2,3-trimethylguanidinium-hydrochlorid (II) mindestens 2 Mol Cyanhydrin-Derivat (III) ein.

In beiden Fällen setzt man pro Mol 1-Amino-2,2,3-trimethyl-guanidinium-hydrochlorid (II) 1-3 Mol, vorzugsweise 1-2,5 Mol Säurebindemittel ein.

Die Reaktionsdurchführung, Aufarbeitung und Isolierung der Reaktionsprodukte erfolgt nach allgemein üblichen Methoden.

Als Reaktionspartner zur Durchführung des erfindungsgemäßen Verfahren (B) kommen übliche anorganische oder organische Säuren in Gegenwart von Wasser in Betracht. Mit Vorzug verwendet man anorganische Protonensäuren, wie insbesondere Salzsäure, Schwefelsäure, Salpetersäure oder Phosphorsäure.

Als Verdünnungsmittel zur Durchführung des erfindungsgemäßen Verfahrens (B) kommen insbesondere mit Wasser mischbare organische Lösungsmittel oder wässrige Systeme infrage. Mit besonderem Vorzug verwendet man einen entsprechenden Überschuß an der als Reaktionspartner eingesetzten wässrigen Säure gleichzeitig als Verdünnungsmittel.

Die Reaktionstemperaturen können bei der Durchführung des erfindungsgemäßen Verfahrens (B) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (B) setzt man pro Mol an 1,6-Dihydro-3-dimethylamino-4-methyl-1,2,4-triazin-Derivat der Formel (Ia) im allgemeinen 1.0 bis 50.0 Mol, vorzugsweise 1.0 bis 30.0 Mol an wässriger Säure ein.

Die Reaktionsdurchführung, Aufarbeitung und Isolierung der Reaktionsprodukte erfolgt nach allgemein

üblichen Methoden (vergleiche auch die Herstellungsbeispiele).

Als Verdünnungsmittel zur Durchführung des erfindungsgemäßen Verfahrens (C) kommen alle unter Hydrierungsbedingungen inerten Solventien in Betracht. Vorzugsweise verwendbar sind gesättigte Kohlenwasserstoffe, wie Petrolether, Pentan, Hexan, Heptan, Cyclohexan; Alkohole, wie Methanol, Ethanol, n-Propanol und i-Propanol; oder Ether, wie Diethylether, Dioxan, Tetrahydrofuran oder Ethylenglykoldimethyl- oder -diethylether, Es kann jedoch auch ohne Verdünnungsmittel gearbeitet werden.

Als Hydrierungskatalysatoren können bei dem erfindungsgemäßen Verfahren (C) alle üblicherweise für derartige Reaktionen verwendbaren Katalysatoren eingesetzt werden. Vorzugsweise verwendet man Raney-Nickel oder Edelmetallkatalysatoren, wie Palladium, Ruthenium, Palladiumoxid, Platin oder Platinoxid, gegebenenfalls auf einem geeigneten Trägerstoff, wie z.B. Kohle oder Siliciumdioxid, Die Reaktionstemperaturen können bei dem erfindungsgemäßen Verfahren (C) in einem größeren Bereich variiert werden. Im allgemeinen arbeitet man bei Temperaturen zwischen 50 °C und 250 °C, vorzugsweise bei Temperaturen zwischen 80 °C und 200 °C.

Das erfindungsgemäße Verfahren (C) kann bei Normaldruck, aber auch bei erhöhtem Druck durchgeführt werden. Bevorzugt wird unter erhöhtem Druck in Gegenwart von Wasserstoff gearbeitet. Im allgemeinen arbeitet man bei Druckverhältnissen zwischen 5 und 300 bar, vorzugsweise zwischen 10 und 200 bar.

Bei der Durchführung des erfindungsgemäßen Verfahrens (C) setzt man auf 1 Mol 3-Dimethylamino-4-methyl-1,2,4-triazin-5(4H)-on der Formel (IV) im allgemeinen 3,0 bis 30 Mol Wasserstoff und 0,001 bis 0,1 Mol, vorzugsweise 0,01 bis 0,1 Mol Katalysator ein. Dabei geht man im allgemeinen so vor, daß man die Verbindungen der Formel (IV) in Gegenwart eines Verdünnungsmittels bei der jeweils gewünschten Temperatur mit Wasserstoff im Autoklaven umsetzt. Die Aufarbeitung geschieht nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Verbindungen mit besonders gutem Erfolg zur Bekämpfung von mono- und dikotylen Unkräutern in mono- und dikotylen Kulturen wie beipielsweise Weizen, Mais oder Soja einsetzen. In entsprechenden Aufwandmengen eignen sich die erfindungsgemäßen Wirkstoffe auch als Baumwolldefoliantien.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFUROH); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [-(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 0-(6-Chlor-3-phenyl-pyridazin-4-yl)-S-octyl-thiolcarbonat (PYRIDATE); 2-[4-(6-Chlorchinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN) sind möglich.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

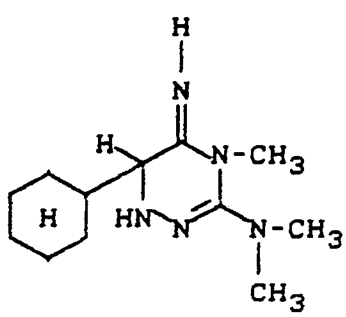

(Verfahren A)

30,5 g (0,2 Mol) 1-Amino-2,2,3-trimethylguanidiniumhydrochlorid und 27,8 g (0,2 Mol) 2-Hydroxy-2-cyclohexylacetonitril werden in 200 ml Methanol bei Raumtemperatur tropfenweise innerhalb 30 Minuten mit 25,3 g (0,25 Mol) Triethylamin versetzt. Anschließend wird die Reaktionsmischung 1 Stunde bei Rückflußtemperatur gerührt. Zur Aufarbeitung wird im Vakuum eingeengt, mit Wasser/Methylenchlorid extrahiert, die organische Phase über Natriumsulfat getrocknet und im Vakuum eingeengt. Zur Reinigung kristallisiert man den Rückstand aus Ligroin um.

Man erhält 15,6 g (33 % der Theorie) an 3-Dimethylamino-4-methyl-6-cyclohexyl-1,6-dihydro-5-imino-1,2,4-triazin vom Schmelzpunkt 138 °C bis 139 °C.

$^1$H-NMR ($CDCl_3$/Tetramethylsilan):

$\delta$ = 1.0-1.8; 2.6; 3.25 ppm.

Beispiel 2

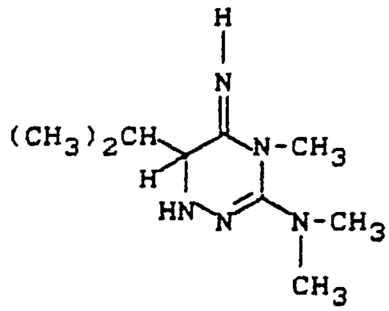

(Verfahren A)

45,75 g (0,3 Mol) 1-Amino-2,2,3-trimethylguanidiniumhydrochlorid und 42,3 g (0,3 Mol) 2-Acetoxy-3-methylbuttersäurenitril werden in 400 ml Methanol mit 27 g (0,5 Mol) Natriummethylat versetzt. Anschließend rührt man 1 Stunde bei Rückflußtemperatur, saugt dann bei Raumtemperatur ausgefallenes Salz ab und engt das Filtrat im Vakuum ein. Der Rückstand wird mit Wasser/Methylenchlorid extrahiert, die organische Phase über Natriumsulfat getrocknet und im Vakuum eingeengt. Zur Reinigung kristallisiert man aus Ligroin um.

Man erhält 26 g (44 % der Theorie) an 3-Dimethylamino-4-methyl-6-isopropyl-1,6-dihydro-5-imino-1,2,4-triazin vom Schmelzpunkt 117 °C bis 119 °C.

$^1$H-NMR ($CDCl_3$/Tetramethylsilan):

$\delta$ = 0.85-0.95; 1.9-2.0; 2.5; 3.1; 5.8; 7.15 ppm.

Beispiel 3:

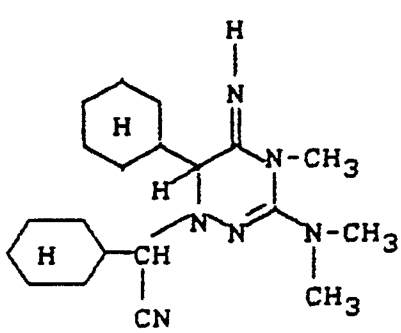

(Verfahren A)

30,5 g (0,2 Mol) 1-Amino-2,2,3-trimethylguanidiniumhydrochlorid und 55,6 g (0,4 Mol) 2-Hydroxy-2-cyclohexylacetonitril werden in 250 ml Methanol 2 Stunden bei Rückflußtemperatur gerührt. Dann gibt man tropfenweise unter Beibehaltung der Rückflußtemperatur innerhalb von 10 Minuten 25,3 g (0,25 Mol)

Triethylamin zu und rührt weitere 2 Stunden bei Rückflußtemperatur. Zur Aufarbeitung wird die erkaltete Lösung im Vakuum eingeengt, der Rückstand mit Wasser/Methylenchlorid extrahiert, die organische Phase über Natriumsulfat getrocknet und im Vakuum eingeengt. Der Rückstand wird durch Säulenchromatographie (Kieselgel; Cyclohexan/Essigester 1:1) gereinigt.

Man erhält 17 g (23,8 % der Theorie) an 2-Cyclohexyl-2-[1-(6-cyclohexyl-3-dimethylamino-5-imino-4-methyl-1,6-dihydro-1,2,4-triazinyl)]-acetonitril als Öl.

$^1$H-NMR ($CDCl_3$/Tetramethylsilan):

$\delta$ = 1.0-2.0; 2.65; 3.25; 3.3-3.35; 3.55-3.6 ppm.

Beispiel 4

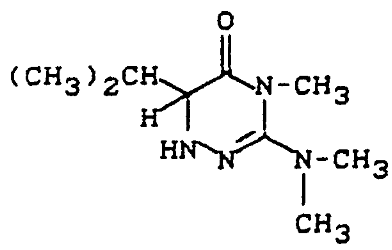

(Verfahren B)

4.6 g (0,023 Mol) 3-Dimethylamino-4-methyl-6-isopropyl-1,6-dihydro-5-imino-1,2,4-triazin werden mit 40 ml 37%iger Salzsäure 40 Minuten bei Rückflußtemperatur gerührt. Nach dem Erkalten verdünnt man mit 40 ml Wasser, stellt mit wässriger Ammoniaklösung einen schwach alkalischen pH-Wert ein und extrahiert mit Methylenchlorid. Die organische Phase wird über Natriumsulfat getrocknet, im Vakuum eingeengt und der Rückstand durch Säulenchromatographie (Kieselgel; Cyclohexan/Essigester 1:1) gereinigt.

Man erhält 2,4 g (51,8 % der Theorie) an 3-Dimethylamino-4-methyl-6-isopropyl-1,6-dihydro-1,2,4-triazin-5-(4H)-on als Öl.

$^1$H-NMR ($CDCl_3$/Tetramethylsilan)

$\delta$ = 1.0-1.05; 1.2-1.3; 2.25-2.35; 2.6; 3.2 ppm.

Beispiel 5:

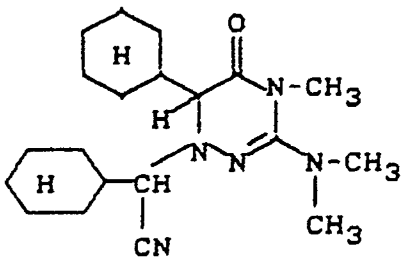

(Verfahren B)

10 g (0.028 Mol) 2-Cyclohexyl-2-[1-(6-cyclohexyl-3-dimethylamino-5-imino-4-methyl-1,6-dihydro-1,2,4-triazinyl)]-acetonitril werden in 100 ml 37%iger Salzsäure 1 Stunde bei Rückflußtemperatur gerührt, anschließend abgekühlt, mit Eiswasser verrührt und mit wässriger Ammoniaklösung auf einen schwach alkalischen pH-Wert eingestellt. Das ausgefallene Produkt wird abgesaugt mit Wasser gewaschen und durch Säulenchromatographie (Kieselgel; Cyclohexan/Essigester 1:1) gereinigt.

Man erhält 5 g (50 % der Theorie) an 2-Cyclohexyl-2-[1-(6-Cyclohexyl-3-dimethylamino-4-methyl-1,6-dihydro-1,2,4-triazin-5-on-yl)]-acetonitril als Öl.

$^1H$-NMR ($CDCl_3$/Tetramethylsilan):
$\delta$ = 1.0-2.1; 2.6; 2.9-2.95; 3.1; 3.15-3.2 ppm.

Beispiel 6:

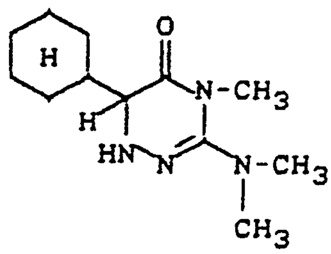

(Verfahren C)

23,6 g (0,1 Mol) 3-Dimethylamino-4-methyl-6-cyclohexyl-1,2,4-triazin-5-on werden in einen 0,3 l-Autoklaven gegeben, mit 190 ml Ethanol und 2 g Pt-Kohle (5%ig) versetzt und 6 Stunden bei 120 °C und 140 bar Wasserstoffdruck hydriert. Zur Aufarbeitung wird abgekühlt, filtriert und im Vakuum eingeengt. Den Rückstand kristallisiert man aus Cyclohexan/Petrolether um.
Man erhält 19,7 g (83 % der Theorie) an 3-Dimethylamino-4-methyl-6-cyclohexyl-1,6-dihydro-1,2,4-triazin-5-(4H)-on vom Schmelzpunkt 90 °C bis 91 °C.
$^1H$-NMR ($CDCl_3$/Tetramethylsilan):
$\delta$ = 1.0-1.8; 2.55; 2.90; 3.05; 6.15 ppm.

In entsprechender Weise und gemäß den allgemeinen Angaben zur Herstellung erhält man die folgenden 1,6-Dihydro-3-dimethylamino-4-methyl-1,2,4-triazin-Derivate der allgemeinen Formel (I):

Tabelle 1

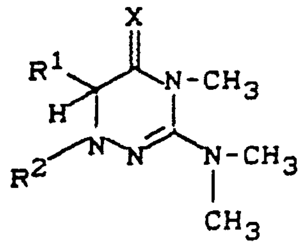

(I)

| Bsp.Nr. | $R^1$ | $R^2$ | X | physikalische Eigenschaften |
|---|---|---|---|---|
| 7 | $-CH_3$ | H | O | Fp.45-46° C |
| 8 | 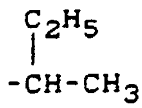 | H | O | Kp.155° C/ 0,1mbar |
| 9 | 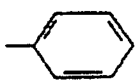 | H | O | Fp.97-98° C |
| 10 | 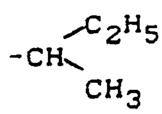 | H | NH | Fp.54-56° C |
| 11 | $-(CH_2)_2-CH_3$ | H | NH | $^1$H-NMR*): 0.95-1.05; 2.60; 3.25; 3.35-3.4 |
| 12 | 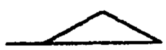 | H | NH | Fp.112-114° C |
| 13 | 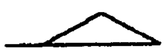 | 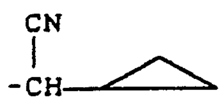 | NH | $^1$H-NMR*): 0.4-0.85; 9.95-1.05; 1.55-1.65; 2.60; 3.35; 3.75-3.80 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen:

Beispiel IV-1:

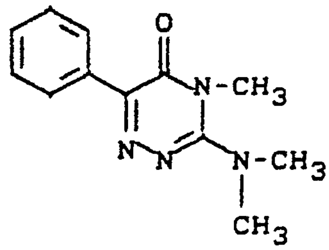

Zu 76,25 g (0,5 Mol) 1-Amino-2,2,3-trimethylguanidiniumhydrochlorid in 1000 ml Methanol werden 100 g (0,6 Mol) Phenylglyoxylsäure-methylester und 101 g (1 Mol) Triethylamin gegebenen. Man rührt 8 Stunden bei Raumtemperatur und eine weitere Stunde bei Rückflußtemperatur, kühlt ab, engt im Vakuum ein und verrührt den Rückstand mit Wasser. Das ausgefallene Produkt wird abgesaugt und aus Cyclohexan/Essigester umkristallisiert.

Man erhält 73,8 g (64,2 % der Theorie) an 3-Dimethylamino-4-methyl-6-phenyl-1,2,4-triazin-5(4H)-on vom Schmelzpunkt 110 °C bis 112 °C.

$^1$H-NMR ($CDCl_3$/Tetramethylsilan):
$\delta$ = 2.97; 3.50; 7.35-7.45; 8.15-8.20 ppm.

Beispiel II-1

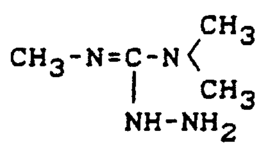 x HCl

Zu 50 g (1 Mol) Hydrazinhydrat in 300 ml Isopropanol tropft man bei 20 °C bis 25 °C unter Rühren innerhalb von 30 Minuten eine Lösung von 78,5 g (0,5 Mol) Chlortrimethylformamidiniumhydrochlorid in 250 ml Isopropanol, rührt nach beendeter Zugabe weitere 30 Minuten bei Raumtemperatur, saugt ausgefallenes Hydrazinhydrochlorid ab, wäscht mit 150 ml Isopropanol nach und engt das Isopropanolfiltrat im Vakuum ein.

Man erhält 70,7 g (93 % der Theorie) an 1-Amino-2,2,3-trimethylguanidiniumhydrochlorid, welches ohne Reinigung weiter umgesetzt wird.

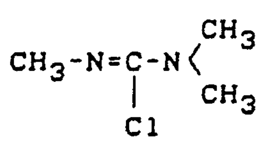 x HCl

In eine Mischung aus 510 g (5 Mol) N,N,N'-Trimethylharnstoff und 3 l Chlorbenzol leitet man bei 80 °C innerhalb von 2,5 Stunden unter Rühren 545 g (5,5 Mol) Phosgen ein und rührt nach beendetem Einleiten weitere 45 Minuten bis zum Ende der Kohlendioxid-Entwicklung bei 80 °C nach. Die Reaktionsmischung wird abgekühlt auf 10 °C, das wasserempfindliche Produkt unter Stickstoff abgesaugt, mit 1 l Chlorbenzol und zweimal mit jeweils 500 ml Petrolether gewaschen und im Vakuum getrocknet.

Man erhält 635,3 g (81 % der Theorie) an Chlortrimethylformamidiniumhydrochlorid vom Schmelzpunkt 76 °C bis 78 °C.

Anwendungsbeispiele

In den folgenden Anwendungsbeispielen wurden die nachstehend aufgeführten Verbindungen als Vergleichssubstanzen eingesetzt:

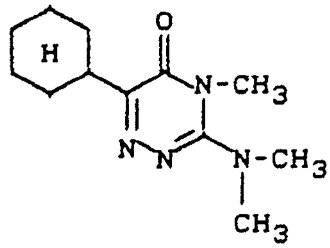

(A)

3-Dimethylamino-4-methyl-6-cyclohexyl-1,2,4-triazin-5-(4H)-on

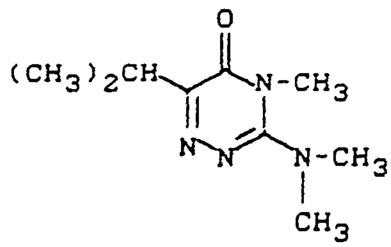

(B)

3-Dimethylamino-4-methyl-6-isopropyl-1,2,4-triazin-5-(4H)-on
[(A) bekannt aus EP 15 452; (B) bekannt aus EP 168 352].

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 8 und 9.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im

Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 8 und 9.

## Patentansprüche

1. 1,6-Dihydro-3-dimethylamino-4-methyl-1,2,4-triazin-Derivate der allgemeinen Formel (I),

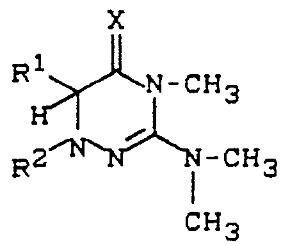

(I)

in welcher

$R^1$ für Alkyl, Cycloalkyl, für gegebenenfalls substituiertes Aralkyl oder für gegebenenfalls substituiertes Aryl steht,

$R^2$ für Wasserstoff oder für einen Rest

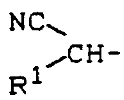

steht, wobei $R^1$ die oben angegebene Bedeutung hat, und

X für Sauerstoff oder eine NH-Gruppe steht.

2. Triazinderivate der allgemeinen Formel (I) gemäß Anspruch 1, dadurch gekennzeichnet, daß darin

$R^1$ für geradkettiges oder verzweigtes Alkyl mit 1 bis 12 Kohlenstoffatomen, für Cycloalkyl mit 3 bis 8 Kohlenstoffatomen, für gegebenenfalls im Arylteil einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen steht, wobei als Arylsubstituenten jeweils infrage kommen:
Halogen, Cyano, Amino, jeweils geradkettiges oder verzweigtes Akyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^2$ für Wasserstoff oder für einen Rest

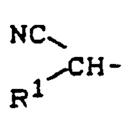

steht, wobei $R^1$ die zuvor angegebene Bedeutung hat, und

X für Sauerstoff oder eine NH-Gruppe steht.

3. Triazinderivate der allgemeinen Formel (I) gemäß Anspruch 1, dadurch gekennzeichnet, daß darin

$R^1$ für geradkettiges oder verzweigtes Alkyl mit 1 bis 6 Kohlenstoffatomen, für Cycloalkyl mit 3

bis 7 Kohlenstoffatomen, für gegebenenfalls im Phenylteil ein- bis dreifach, gleich oder verschieden substituiertes Phenylalkyl mit 1 bis 4 Kohlenstoffatomen im geradkettigen oder verzweigten Alkylteil oder für gegebenenfalls ein- bis dreifach, gleich oder verschieden substituiertes Phenyl steht, wobei als Substituenten jeweils infrage kommen:
Fluor, Chlor, Brom, Amino, Methyl, Ethyl, n- oder i-Propyl, Methoxy, Ethoxy, n- oder i-Propoxy, Methylthio, Trifluormethyl, Trifluormethoxy oder Trifluormethylthio,

$R^2$ für Wasserstoff oder für einen Rest

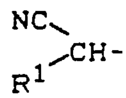

steht, wobei $R^1$ die zuvor angegebene Bedeutung hat, und

X für Sauerstoff oder eine NH-Gruppe steht.

**4.** Triazinderivate der allgemeinen Formel (I) gemäß Anspruch 1, dadurch gekennzeichnet, daß darin

$R^1$ für Methyl, Ethyl, n- oder i-Propyl, n-, i-, s- oder t-Butyl, für Cyclopropyl, Cyclopentyl, Cyclohexyl, für gegebenenfalls einfach oder zweifach im Phenylteil substituiertes Benzyl oder für gegebenenfalls einfach oder zweifach substituiertes Phenyl steht, wobei als Substituenten jeweils infrage kommen:
Amino, Fluor, Chlor, Brom, Methyl, Ethyl, Methoxy oder Ethoxy,

$R^2$ für Wasserstoff steht und

X für Sauerstoff oder eine NH-Gruppe steht.

**5.** Verfahren zur Herstellung von 1,6-Dihydro-3-dimethylamino-4-methyl-5-imino-1,2,4-triazin-Derivaten der Formel (Ia),

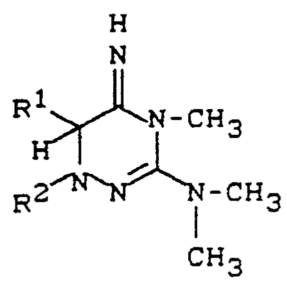

(Ia)

in welcher

$R^1$ für Alkyl, Cycloalkyl, für gegebenenfalls substituiertes Aralkyl oder für gegebenenfalls substituiertes Aryl steht und

$R^2$ für Wasserstoff oder für einen Rest

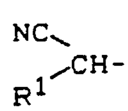

steht, wobei $R^1$ die oben angegebene Bedeutung hat,

dadurch gekennzeichnet, daß man 1-Amino-2,2,3-trimethylguanidiniumhydrochloridder Formel (II)

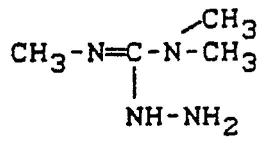 x HCl (II)

mit Cyanhydrin-Derivaten der Formel (III),

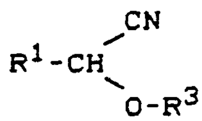 (III)

in welcher

$R^1$ die oben angegebene Bedeutung hat und

$R^3$ für Wasserstoff oder eine Acetylgruppe steht,

im Molverhältnis 1:1 oder im Molverhältnis 1:2, gegebenenfalls in Gegenwart eines Verdünnungsmittels und gegebenenfalls in Gegenwart eines Säurebindemittels umsetzt, wobei man bei einem Molverhältnis der Ausgangsstoffe (II) : (III) von 1:1 Endprodukte der Formel (Ia), worin $R^2$ für H steht, und bei einem Molverhältnis (II) : (III) von 1:2 Endprodukte der Formel (Ia), worin

$R^2$ für einen Rest

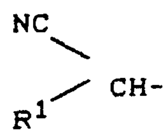

steht, erhält.

6. Verfahren zur Herstellung von 1,6-Dihydro-3-dimethylamino-4-methyl-1,2,4-triazin-5(4H)-on-Derivaten der Formel (Ib),

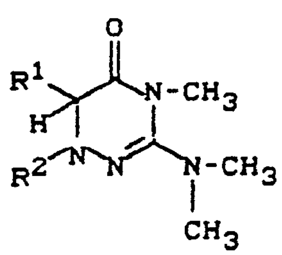 (Ib)

in welcher

$R^1$ für Alkyl, Cycloalkyl, für gegebenenfalls substituiertes Aralkyl oder für gegebenenfalls substituiertes Aryl steht und

$R^2$ für Wasserstoff oder für einen Rest

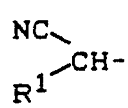

steht, wobei $R^1$ die zuvor angegebene Bedeutung hat,

dadurch gekennzeichnet, daß man (die nach Anspruch 5 erhältlichen) 1,6-Dihydro-3-dimethylamino-4-methyl-5-imino-1,2,4-triazin-Derivate der Formel (Ia),

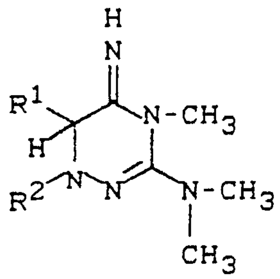

(Ia)

in welcher
$R^1$ und $R^2$ die oben angegebene Bedeutung haben,
mit wässriger Säure gegebenenfalls in Gegewart eines Verdünnungsmittels umsetzt.

7. Verfahren zur Herstellung von 1,6-Dihydro-3-dimethylamino-4-methyl-1,2,4-triazin-5(4H)-on-Derivaten der Formel (Ic),

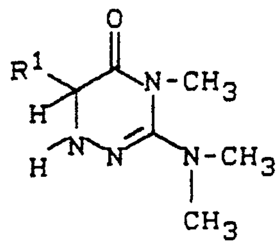

(Ic)

in welcher
$R^1$ für Alkyl, Cycloalkyl, für gegebenenfalls substituiertes Aralkyl oder für gegebenenfalls substituiertes Aryl steht,
dadurch gekennzeichnet, daß man 3-Dimethylamino-4-methyl-1,2,4-triazin-5(4H)-on der Formel (IV),

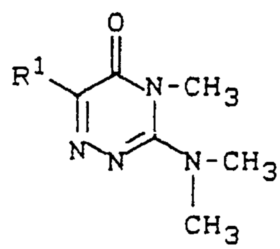

(IV)

in welcher
$R^1$ die oben angegebene Bedeutung hat,
mit Wasserstoff in Gegenwart eines Hydrierungskatalysators und gegebenenfalls in Gegenwart eines Verdünnungsmittels umsetzt.

8. Herbizide Mittel, gekennzeichnet durch einen Gehalt an mindestens einem 1,6-Dihydro-3-dimethylamino-4-methyl-1,2,4-triazin-Derivat der Formel (I) gemäß Anspruch 1.

9. Verwendung von 1,6-Dihydro-3-dimethylamino-4-methyl-1,2,4-triazin-Derivaten der Formel (I) gemäß Anspruch 1 zur Bekämpfung von Unkräutern.

10. Verfahren zur Herstellung von herbiziden Mitteln, dadurch gekennzeichnet, daß man 1,6-Dihydro-3-dimethylamino-4-methyl-1,2,4-triazin-Derivate der Formel (I) gemäß Anspruch 1 mit Streckmitteln

und/oder oberflächenaktiven Mitteln vermischt.

Europäisches Patentamt

# EUROPÄISCHER RECHERCHENBERICHT

Nummer der Anmeldung

**EP 91 10 7256**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D,D,D-,D | EP-A-0 015 452 (BAYER AG)<br>* Ansprüche 1, 3-6 & DE-A-2908963 & DE-A-2908964 & DE-A-2938384 *<br>– – – | 1,8-10 | C 07 D 253/06<br>A 01 N 43/707 |
| A | EP-A-0 130 519 (BAYER AG)<br>* Ansprüche 1, 4-6 *<br>– – – | 1,8-10 | |
| A,D | EP-A-0 168 352 (CIBA-GEIGY AG)<br>* Ansprüche 1, 10, 20-22 *<br>– – – – – | 1,8-10 | |

RECHERCHIERTE SACHGEBIETE (Int. Cl.5)

C 07 D 253/00
A 01 N 43/00

Der vorliegende Recherchenbericht wurde für alle Patentansprüche erstellt

| Recherchenort | Abschlußdatum der Recherche | Prüfer |
|---|---|---|
| Berlin | 09 August 91 | VAN AMSTERDAM L.J.P. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument